# EUROPEAN PATENT SPECIFICATION

(11) **EP 2 067 496 B1**
(45) Date of publication and mention of the grant of the patent: **27.04.2011**
(21) Application number: 09156072.2
(22) Date of filing: 23.06.2003
(51) Int. Cl.: A61M 5/32, A61M 5/315

(54) **Medical injector**
Medizinisches Injektionsgerät
Injecteur médical

(30) Priority: 03.02.2003 GB 0302393
(43) Date of publication of application: 10.06.2009
(62) Divisional of application: 03760811.4
(73) Proprietor: Liversidge, Barry Peter, Colchester Essex C04 5PE (GB)
(72) Inventor: Liversidge, Barry Peter, Colchester Essex C04 5PE (GB)
(74) Representative: Gillam, Francis Cyril

(56) References cited:
- EP-A1- 0 516 473
- WO-A-03/097133
- WO-A-2004/054645
- WO-A2-01/62319
- US-A- 3 742 948
- US-A- 5 298 024
- US-A- 5 616 128

## Description

This invention relates to a mechanism for performing an injection having a needle intended for penetration of a human or animal body, or for other medical uses such as the penetration of a pierceable membrane of an intravenous medication system. For convenience, in the following all such medical uses will be described simply as the penetration of a body, even though specific embodiments may be intended for other medical uses.

Fluids of various kinds may be administered to a human or animal body by means of a hollow needle in conjunction with a source of the required fluid. For example, such a needle may be mounted on a syringe pre-filled with a liquid drug, the needle being used to penetrate the body at the site at which the drug is to be received, the drug then being expelled from the syringe by applying force to the rear end of the syringe plunger.

US-A-6102896 discloses a disposable injector device that is operable by hand-force and has a plunger, a barrel and snap means, such as break-tabs or a snap-ring, to release the plunger abruptly when a snap-point is reached. In some embodiments, the device includes inter-engaging cap and base parts. The cap part has an integral plunger portion and the base part defines a bore that accommodates the barrel; a needle may be connected to a front end of the barrel. Operation of the cap part causes the plunger portion to move with respect to the barrel to drive the barrel along the bore for penetrating the needle into a patient's skin and to expel liquid medicament contained within the barrel through the needle into the patient's body. The snap means are provided between the cap part and the base part, and also between the barrel and the base part and are adapted to break or release when the snap-point is reached. The snap means release abruptly as hand-force reaches the snap-point causing the medicament to be expelled rapidly from the injector. The break-tabs between the cap part and the base part and between the barrel and the base part may break simultaneously or may have different break-points

It will be appreciated that a successful injection requires a correct sequence of events: firstly the insertion of the needle into a body to the correct depth followed by the complete delivery of the drug through the hollow needle and into the body. This required sequence has been attained by a variety of prior art devices, such as those described in the following summaries.

US-A-2001/0005781 describes a medical injector for use with a pre-filled syringe having a needle secured to the forward end thereof and in which there is a mechanism arranged selectively to drive forwardly the syringe and needle so as to penetrate a body and then to apply a force to the plunger so as to expel the drug from the syringe, thus completing the correct injection sequence. The medical injector has a safety device on the forward end thereof and including a sleeve slidable with respect to the needle, to protect the needle both before and after an injection. In operation, the medical injector mechanism is arranged push the syringe and needle forwardly while preventing forward movement of the plunger and piston until the needle has sufficiently penetrated a patient, whereafter the drug is dispensed through the needle. This proper sequencing of the injection is controlled by a structure within the injector that only allows movement of the syringe plunger and piston when the whole syringe has moved forwardly by a sufficient extent.

US-A-3797489 discloses a similar arrangement for use with a pre-filled syringe wherein spring power drives an ampoule and cannula to insert the cannula in a patient and then inject the drug. The sequencing is again performed by a structure within the injector and which allows forward movement of the plunger only when the syringe has moved forwardly a sufficient extent.

Further examples of similar injectors including structures to ensure proper sequencing as aforesaid are to be found in US-A-3742948, US-A-2752918 , US-A-9055362 , GB-A-2388033 , EP1364667-A2 and WOA2005/002653.

All of the prior art devices as described in the various specifications aforesaid use structures which are of varying degrees of complexity, to achieve a properly sequenced injection. The problem of this sequencing has been long understood - as evidenced by US-A-2752918, filed on 1st May 1952.

Despite this, many injector designs ignore the problem of sequencing; WO-A-03/013632 describes an medical injector having a mechanism which acts directly on a syringe plunger so as to drive the piston and thereby the entire syringe, needle and carrier forwards in relation to an external barrel by virtue of "the effective solidity of the dose" within a capsule. Thereafter, further pressing on the plunger continues to eject the dose, but as is well known and understood, medicament inevitably will leak through the needle during the first stage of operation when pressure is applied to the plunger to drive forwardly the entire syringe and carrier. Since liquid medicament is not truly solid, there will be unwanted loss of drug before the needle has entered the body.

WO-A-03/097133 discloses an injection device with an automatically retractable needle. The needle extends forwardly from a capsule that can slide longitudinally within a barrel-like body, a relatively weak spring normally maintaining the capsule and needle retracted. A more powerful spring acts oppositely on a plunger which, when released, shoots the capsule forward by acting on the liquid therein, and then forces the liquid out through the projecting needle. At the end of the forward stroke the plunger and capsule are decoupled and the weak spring returns the exhausted capsule and its needle to the retracted position.

US-A-3742948 discloses an hypodermic syringe comprising a liquid container and a needle connected thereto at one end, which assembly is incorporated in a housing, the liquid container being bounded at the other end by a piston, and an operating mechanism being present to exert a force on the piston, one or more blocking elements being present for locking the operating mechanism or the piston against movement relative to the liquid container, the housing showing a diameter transition such that when the blocking elements pass along it, they are moved radially so that the blocking is removed and the piston penetrates into the liquid container so that the liquid flows out through the needle.

WO-A-01162319 discloses syringes including various forms of a releasable stop adapted to limit selectively the travel of the syringe plunger within the syringe barrel, such that a first amount of therapeutic substance may be discharged from the syringe while a residual supply of the therapeutic substance remains in the syringe. Movement of the plunger is permitted to displace the residual supply of fluid from the barrel. The syringes of WO-A-01/62319 find particular application as diluent syringes in a wet/dry drug delivery syringe system having two cooperating syringe barrels in which the residual supply of diluent in the diluent syringe may be used to flush additional and otherwise wasted drug from a drug syringe barrel. In one embodiment the releasable stop comprises a leaf spring formed integrally with the plunger shaft and cooperating with an annular ledge formed on the interior of the syringe barrel. A recess is formed in the shaft to accommodate the leaf spring and permit radially inward displacement thereof relative to the plunger shaft.

As is clear from the wealth of prior art, there is a continuing need for a mechanism for performing an injection that is able to achieve proper sequencing for an injection, of firstly inserting the needle fully and then dispensing the drug.

According to this invention there is provided a mechanism for performing an injection, the mechanism comprising a syringe, a plunger and a sleeve, wherein:
- the syringe has a needle with a sharp tip at the forward end thereof, and the body of the syringe has a bore housing a piston so that a medicament contained within the syringe can be expelled through the needle by moving the piston;
- the syringe is disposed within the sleeve, which has a forward end and is slideable rearwardly relative to the syringe to allow the needle of the syringe to project from said forward end; and:
- said plunger is provided with protuberances disposed partway along the length thereof, and in the region of the protuberances the plunger has a through-slot to enable radially inward movement of the protuberances, so that the protuberances define a stop position for the plunger on being moved into the bore by the application of axial pressure to the remote end of the plunger, whereby when the protuberances are about to enter the rear end of the syringe body, said pressure moves the syringe relative to the sleeve to cause the needle to project from the forward end of the sleeve, and an increase in force is then momentarily required to move the plunger deeper into the syringe body, thus moving the piston and expelling the medicament from within the syringe.

It will be appreciated that this invention allows the proper sequencing of an injection when the syringe is slidable relative to an external sleeve such that pressure applied to the rear end of a plunger will move the syringe and needle forwardly relative to the sleeve to allow the needle fully to penetrate a body whereafter increased force on the plunger will move the plunger past the stop position, the protuberance moving inwardly to permit this forward movement of the plunger, and dispense the medicament from the syringe body through the needle. Thus the protuberances on the plunger enable the medical injector automatically to sequence a successful injection.

The plunger of the preferred embodiment may have an X-shaped cross-section that defines a plurality of arms, wherein the outer edge of each arm of the cross-section is provided with a protuberance.

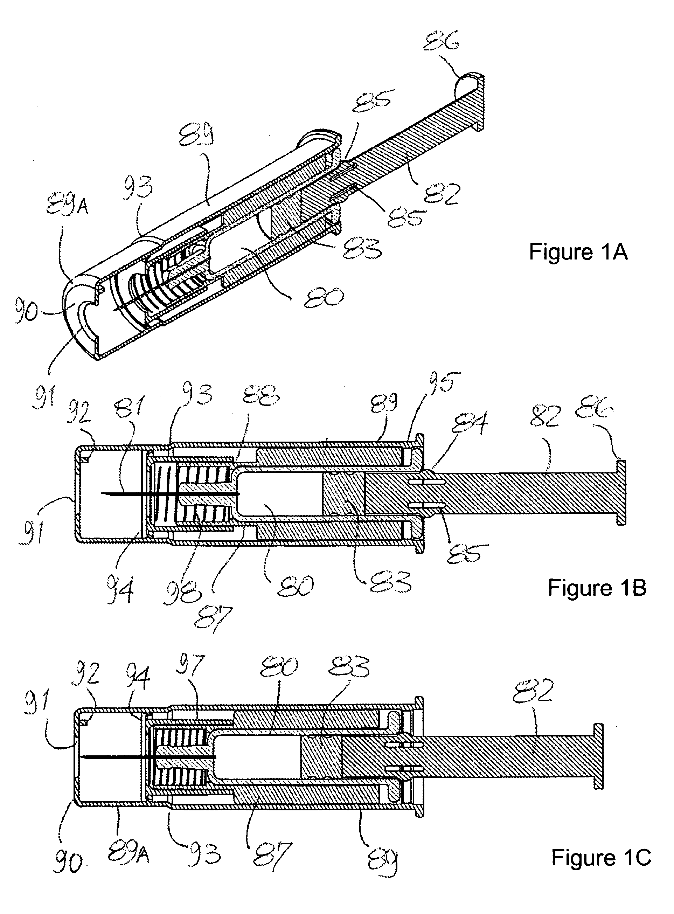

By way of example only, one embodiment of this invention will now be described in detail, with reference to the accompanying drawings, showing the embodiment in various settings. In the drawings, Figures 1A to 1J show a pre-filled syringe or a syringe to be filled from a vial of medicament, in varying stages of the use of the device.

### [Note: The suffix letter I is not used in the identification of the Figures to avoid confusion with the numeral 1]

In the following description of the embodiment of this invention, the terms front, forward, and so on are used to refer to that end of the needle assembly whereat the sharp tip of the needle is located and also to the direction of insertion of the needle into a body. Conversely, the terms rear, rearwardly and so on are used to refer to the other end of the needle assembly, to which is connected other equipment such as a syringe or a blood collection system, and also to the direction of removal of a needle from a body.

### Figures 1A to 1J

The arrangement shown in the drawings uses a syringe having a body 80 fitted with a needle 81 in the course of manufacture. The syringe has a plunger 82 with a piston 83 within the bore of the syringe, so that liquid may be drawn into the syringe through the needle 81 by withdrawing the plunger 82 from its fully inserted position, the medicament subsequently being expelled through the needle 81 by depressing that plunger.

The plunger 82 has an X-shaped cross-section and differs from the plunger of a conventional syringe in that the outer edge of each arm of the X-shaped cross-section is provided with a protuberance 84, disposed approximately one quarter of the way along the length of the plunger, from the piston end. In the region of each protuberance, the respective arm has a through-slot 85 to enable radially inward movement of the protuberances. The protuberances 84 define a stop position for the plunger on being moved into the bore by the application of axial pressure to the remote end 86 of the plunger. When the protuberances reach the rear end of the syringe body, an increased force is momentarily required to move the plunger deeper into the syringe body.

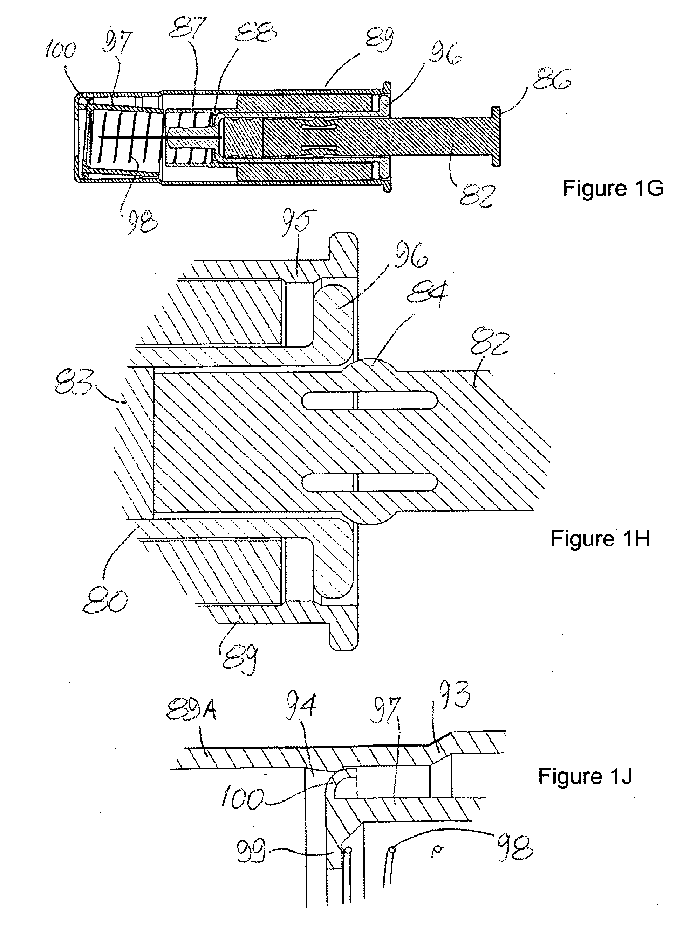

The syringe described above is provided with a safety arrangement which comprises a tubular support 87 having a bore in which the syringe body 80 is snugly received. The needle is thus indirectly carried by the support, through the syringe itself. Formed within that bore is an internal rib 88 which limits the movement of the syringe body into the bore. The part of the support 87 which overlies the syringe body has a greater wall thickness and slidably carries a sleeve 89. The forward end 89A of the sleeve has an internal radial flange 90 formed with a central hole 91 through which the needle 81 may project, the flange being provided with an upstand 92 which projects internally of the sleeve towards the syringe, the upstand having a relatively small arcuate extent, typically of only a few degrees. Partway along the length of the sleeve 89, an annular shoulder 93 is formed by a change in the internal and external diameters of the sleeve and between that shoulder 93 and the flange 90, there is formed an inwardly-projecting annular rib 94 (Figure 1 J). A further internal rib 95 (Figure 1H) is formed at the rearward end of the sleeve, over which an out-turned flange 96 at the rear end of the syringe body must ride to permit the sleeve to slide rearwardly from that position shown in Figures 1A, 1B and 1H.

A tubular blocking member 97 is slidably carried on the forward end portion of the support 87 and is urged forwardly by a helical compression spring 98, acting between the internal rib 88 of the support and an internal flange 99 formed at the forward end of the blocking member 97. Externally, the blocking member 97 has at its forward end an outwardly-projecting flexible lip 100 slidable within the smaller diameter portion of the sleeve 89 but movable over the internal rib 94 of the sleeve only when an increased force is applied to the sleeve, relative to the blocking member. This is shown on an enlarged scale in Figure 1J.

The starting position is shown in Figures 1A, 1B, 1H and 1J, with the safety arrangement set ready for use, though the plunger will be fully forward, to permit filling of the syringe. Alternatively, the syringe could be pre-filled, in which case the plunger will be set as shown, and the filling step will be omitted.

If the syringe is to be filled, the nose part of a phial (not shown) of medicament is inserted into the hole 91 at the forward end of the sleeve 89 and is pushed gently on to the needle 81, moving the sleeve rearwardly with respect to the syringe by riding the further rib 95 of the sleeve over the out-turned flange 96 of the syringe body 80. During this, the blocking member 97 moves rearwardly, simultaneously with the sleeve, against the action of spring 98. The combined force of the spring 98 acting on the blocking member 97 and the force required to ride the further rib 95 over the out-turned flange 96 should be less than that required to move the lip 100 of the blocking member 97 over the rib 94 of the sleeve. As such, during the phial-filling operation, the blocking member 97 remains with its lip 100 rearward of rib 94 of the sleeve (Figure 1 C).

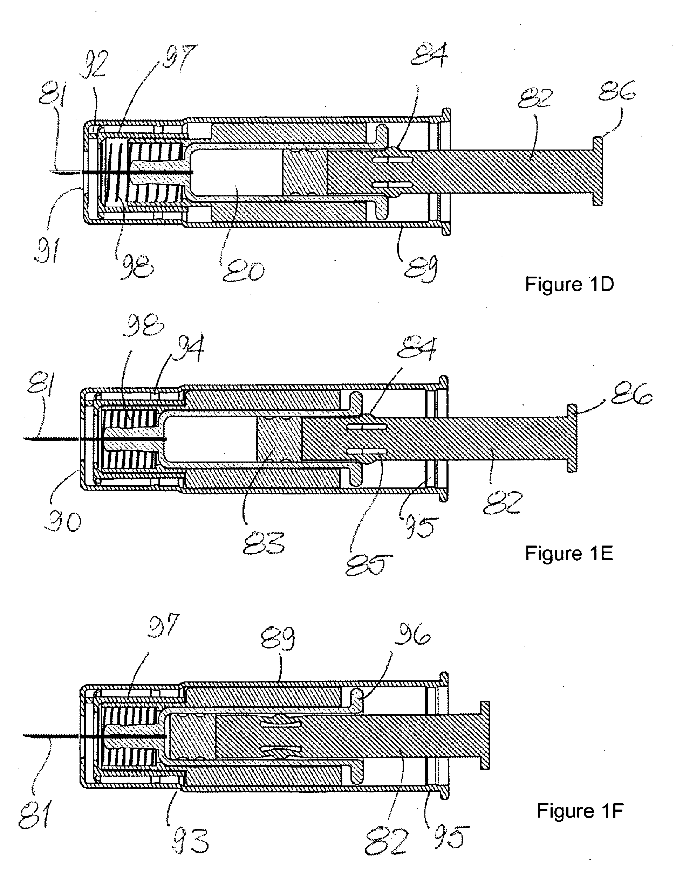

Following charging of the syringe and then the removal of the phial, the mechanism is ready for performing an injection. The operator applies a gentle force on the remote end 86 of the plunger by applying a reaction to the sleeve 89 and this has the effect of moving the plunger forwardly until the protuberances 84 are about to enter the syringe body, and also of pulling the sleeve rearwardly, to cause the needle 81 to project from the forward end of the sleeve. However, this can be achieved only by having the lip 100 of the blocking member 97 ride over the rib 94 of the sleeve 89 andso moving forwardly towards the flange 90 of the sleeve, as shown in Figure 1D. Rearward movement of the sleeve may continue until the shoulder 93 engages that part of the support having a thickened wall thickness as shown in Figure 1E. The needle 81 is then projecting beyond the flange 90 to its greatest possible extent.

The assembly is used in this condition to perform an injection, firstly by pushing the needle 81 into a body at the injection site and then pushing the plunger fully forwardly, the protuberances 84 moving inwardly to permit this, as shown in Figure 1F, The condition of Figure 1E could instead be achieved by using the syringe with the connected assembly to perform a stabbing motion against a body, so that the engagement of the flange 90 at the forward end of the sleeve moves the sleeve rearwardly with respect to the syringe.

On removing the syringe assembly from a body, by pulling rearwardly on the plunger and releasing the sleeve, or by pulling on the sleeve and releasing the plunger, the spring 98 will cause relative separation of the forward end of the sleeve and the support 87, the spring acting on the flange 99 of the blocking member 97 to maintain contact between the forward end of the blocking member and upstand 92. Eventually, the separation will be so great that the blocking member comes free of the support 87 and the spring force acting on the blocking member will allow it to cant over so that its axis lies at an acute angle to the axis of the sleeve and support member - Figure 1G. When in this position, the blocking member 97 lies between the flange 90 of the sleeve and the forward end of the support 87 and so physically blocks subsequent rearward movement of the sleeve 89, with respect to the support and syringe.

When in the setting of Figure 1G, the needle is securely protected against exposure. Having regard to the tubular nature of the blocking member 97, a very high force must be applied to the sleeve 89 in order to expose the needle, in effect either destroying the sleeve or the blocking member.

The respective sleeves may be made transparent, translucent or provided with a transparent or translucent window. By manufacturing the blocking member from a strongly-coloured material, the position of the blocking member within the sleeve will readily be discernible. Then, when the safety device has been used and the blocking member is fully forward, this will immediately be apparent on looking at the assembly.

## Claims

1. A mechanism for performing an injection, the mechanism comprising a syringe, a plunger (82) and a sleeve (89), wherein:
- the syringe has a needle (81) with a sharp tip at the forward end thereof, and the body (80) of the syringe has a bore housing a piston (83) so that a medicament contained within the syringe can be expelled through the needle (81) by moving the piston; and
- the syringe is disposed within the sleeve (89), which has a forward end (89A) and is slideable rearwardly relative to the syringe to allow the needle of the syringe to project from said forward end; **characterised in that**:
- said plunger (82) is provided with protuberances (84) disposed partway along the length thereof, and in the region of the protuberances the plunger has a through-slot (85) to enable radially inward movement of the protuberances, so that the protuberances (84) define a stop position for the plunger on being moved into the bore by the application of axial pressure to the remote end (86) of the plunger, whereby when the protuberances are about to enter the rear end (96) of the syringe body, said pressure moves the syringe relative to the sleeve (89) to cause the needle to project from the forward end (89A) of the sleeve, and an increase in force is then momentarily required to move the plunger deeper into the syringe body, thus moving the piston and expelling the medicament from within the syringe.

2. A mechanism as claimed in claim 1, wherein the plunger (82) has an X-shaped cross-section that defines a plurality of arms, wherein the outer edge of each arm of the cross-section is provided with a protuberance (84).

3. A mechanism as claimed in claim 1, wherein the protuberances (84) are disposed approximately one quarter of the way along the length of the plunger (82) from the piston end.

4. A mechanism as claimed in any of the preceding claims, wherein the syringe body (80) has the needle (81) fitted in the course of manufacture.

## Patentansprüche

1. Mechanismus zum Durchführen einer Injektion mit einer Spritze, einem Stempel (82) und einer Hülse (89), wobei
- die Spritze eine Nadel (81) mit einer scharfen Spitze an ihrem vorderen Ende hat, und der Körper (80) der Spritze eine Bohrung aufweist, die einen Kolben (83) aufnimmt, so dass ein in der Spritze enthaltenes Medikament mittels Bewegen des Kolbens durch die Nadel (81) ausgestoßen werden kann; und
- die Spritze in der Hülse (89) angeordnet ist, welche ein vorderes Ende (89A) hat und relativ zu der Spritze rückwärts bewegbar ist, um der Nadel der Spritze ein Überstehen über das vordere Ende zu erlauben; **dadurch gekennzeichnet, dass**:
- der Stempel (82) auf einem Teil des Weges entlang dessen Länge mit Vorsprüngen (84) versehen ist und im Bereich der Vorsprünge eine Durchgangsöffnung (85) zum Ermöglichen einer radial einwärts gerichteten Bewegung der Vorsprünge aufweist, so dass die Vorsprünge (84) eine Anschlagsposition für den Stempel beim in die Bohrung mittels Beaufschlagen des fernen Endes (86) des Stempels mit einem axialen Druck bewegt werden definieren, wobei der Druck, wenn die Vorsprünge dabei sind, in das rückwärtige Ende (96) des Spritzenkörpers einzutreten, die Spritze relativ zu der Hülse (89) bewegt, um die Nadel zu einem Überstehen über das vordere Ende (89A) der Hülse zu veranlassen, und ein Ansteigen der Kraft dann momentan erforderlich ist, um den Stempel tiefer in den Spritzenkörper hinein zu bewegen, wodurch der Kolben bewegt und das Medikament aus dem Inneren der Spritze ausgestoßen wird.

2. Mechanismus nach Anspruch 1, **dadurch gekennzeichnet, dass** der Stempel (82) einen X-förmigen Querschnitt aufweist, der eine Vielzahl von Armen definiert, wobei der Außenrand jedes Armes des Querschnittes einen Vorsprung (84) aufweist,

3. Mechanismus nach Anspruch 1, **dadurch gekennzeichnet, dass** die Vorsprünge (84) etwa auf einem Viertel des Weges entlang der Länge des Stempels (82) von dessen kolbenseitigem Ende angeordnet sind.

4. Mechanismus nach einem der vorhergehenden Ansprüche, **dadurch gekennzeichnet, dass** die Nadel (81) im Zuge der Herstellung an dem Spritzenkörper (80) befestigt ist.

## Revendications

1. Mécanisme pour réaliser une injection, ledit mécanisme comprenant une seringue, un plongeur (82) et un manchon (89), dans lequel:
- la seringue possède une aiguille (81) ayant à son extrémité avant une pointe aiguisée, et le corps de la seringue possède un alésage recevant un piston (83) de manière à ce qu'un médicament contenu dans le seringue puisse être expulsé à travers l'aiguille (81) en déplaçant le piston; et
- la seringue est disposée à l'intérieur du manchon (89), qui possède une extrémité avant (89A) et qui peut glisser vers l'arrière relativement à la seringue pour permettre à l'aiguille de la seringue de dépasser ladite extrémité avant; **caractérisée en ce que**
- ledit plongeur (82) est muni de protubérances (84) disposées à mi-chemin sur la longueur de celui-ci, et dans la région des protubérances le plongeur possède une fente traversante (85) afin de permettre un mouvement radialement vers l'intérieur des protubérances, de manière à ce que les protubérances (84) définissent une position de butée pour le plongeur lorsqu'elles sont déplacées vers l'intérieur de l'alésage par l'application d'une pression axiale à l'extrémité distante (86) du plongeur, de sorte que lorsque les protubérances sont sur le point de pénétrer dans l'extrémité arrière (96) du corps de seringue, ladite pression déplace la seringue relativement au manchon (89) pour faire en sorte en que l'aiguille dépasse l'extrémité avant (89A) du manchon, et une augmentation de la force est momentanément nécessaire pour déplacer le plongeur plus profondément dans le corps de seringue, déplaçant ainsi le piston et expulsant le médicament de l'intérieur de la seringue.

2. Mécanisme tel que revendiqué dans la revendication 1, dans lequel le plongeur (82) possède une section en X qui définit une pluralité de bras, dans lequel le bord extérieur de chaque bras dans la section est muni d'une protubérance (84).

3. Mécanisme tel que revendiqué dans la revendication 1, dans lequel les protubérances (84) sont disposées sur la longueur du plongeur (82) à environ un quart de la distance en partant de l'extrémité avec le piston.

4. Mécanisme tel que revendiqué dans l'une des revendications précédentes, dans lequel le corps de seringue (80) possède une aiguille (81) assemblée lors de la fabrication.
